# EUROPÄISCHE PATENTSCHRIFT

(11) **EP 0 806 956 B1**
(45) Veröffentlichungstag und Bekanntmachung des Hinweises auf die Patenterteilung: **28.08.2002**
(21) Anmeldenummer: 96901696.3
(22) Anmeldetag: 31.01.1996
(51) Int. Cl.: A61K 31/70, A61P 35/00

(54) **VERWENDUNG VON 5' SUBSTITUIERTEN NUKLEOSIDEN ZUR HEMMUNG VON RESISTENZBILDUNG BEI DER ZYTOSTATIKABEHANDLUNG**
USE OF 5'SUBSTITUTED NUCLEOSIDES TO PROVIDE RESISTANCE IN CYTOSTATIC TREATMENT
UTILISATION DE NUCLEOSIDES SUBSTITUES EN POSITION 5' POUR ATTENUER LES PHENOMENES DE RESISTANCE LORS DE TRAITEMENTS PAR AGENTS CYTOSTATIQUES

(30) Priorität: 01.02.1995 DE 19503152; 08.12.1995 DE 19545892
(43) Veröffentlichungstag der Anmeldung: 19.11.1997
(73) Patentinhaber: RESprotect GmbH, 01307 Dresden (DE)
(72) Erfinder: FAHRIG, Rudolf, D-30657 Hannover (DE); STEINKAMP-ZUCHT, Angela, Dr., D-30625 Hannover (DE)
(74) Vertreter: Pfenning, Meinig & Partner
(86) Internationale Anmeldenummer: DE9600169
(87) Internationale Veröffentlichungsnummer: WO96023506

(56) Entgegenhaltungen:
- EP-A- 0 488 718
- BE-A- 640 236
- GB-A- 1 473 148
- INDIAN JOURNAL OF EXPERIMENTAL BIOLOGY, Bd. 22, Nr. 6, 1984, Seiten 333-334, XP000574699 CHAKRABARTY, MISHRA: "LOSS OF PLASMID LINKED DRUG RESISTANCE AFTER TREATMENT WITH IODO-DEOXYURIDINE"
- TODAY'S LIFE SCIENCE, Bd. 2, Nr. 9, 1990, Seiten 32-38 80, XP000575102 S. LOCARNINI: "HEPATITIS B ANTIVIRAL THERAPY"
- EUROPEAN JOURNAL OF CANCER & CLINICAL ONCOLOGY, Bd. 20, Nr. 4, 1984, Seiten 471-476, XP000575098 WILDIERS, DE CLERCQ: "ORAL (E)-5-(2-BROMOVINYL)-2'-DEOXYURIDINE TREATMENT OF SEVERE HERPES ZOSTER IN CANCER PATIENTS"
- CANCER RESEARCH, Bd. 54, Nr. 10, 1994, Seiten 2701-2706, XP000574738 CHI, KUNUGI, KINSELLA: "IODODEOXYURIDINE CHEMOSENSITIZATION OF CIS-DIAMMINEDICHLOROPLATINUM(II) IN HUMAN BLADDER CANCER CELLS"
- J. CELL. PHARMACOL., Bd. 1, Nr. 1, 1990, Seiten 2-7, XP000575045 WROBLEWSKI, HACKER: "THE POSSIBLE ROEL OF ALTERED NUCLEOTIDE METABOLISM IN CISPLATIN RESISTANCE"

## Beschreibung

Das Auftreten von "Arzneimittel-Resistenz" ist der Hauptgrund für Fehlschläge in der Krebs-Chemotherapie. Tumoren, welche anfangs empfindlich auf Zytostatika reagieren, erholen sich sehr häufig nach einer gewissen Behandlungszeit und sind dann resistent gegenüber der Wirkung verschiedenartiger antineoplastischer Arzneimittel. Obwohl das Problem der "Arzneimittel-Resistenz" schon seit 1948, dem Beginn der Krebstherapie mit Zytostatika, bekannt ist, wurde bis heute kein Weg gefunden, die Resistenzbildung zu verhindern. Charakteristisch für alle bisher untersuchten hochresistenten Tumoren und Zellinien ist die Amplifikation (Vervielfachung) einer kleinen Gruppe von Genen. Als Folge dieser DNA- oder Genamplifikation kann eine erhöhte Expression dieser Gene nachgewiesen werden, welche zur Resistenz gegenüber dem Arzneimittel führt. Als Folge dieser DNA-Amplifikation kann eine erhöhte Expression verschiedener Gene nachgewiesen werden. Schutzproteine, die zur Ausschleusung von Giftstoffen aus der Zelle dienen, können so vermehrt gebildet werden (P-Glycoprotein). Dieser Effekt wird auch als "multi-drug-resistance" (MDR) bezeichnet.

Zusätzlich zur MDR korreliert der Amplifikationsgrad gewisser Gene, vor allem gewisser Onkogene, mit dem Grad der Malignität. So sind die Überlebenschancen bei einer Amplifikation von ERVV2, RASKI, INT3, HST1, MYC und KSRAM beim Magenkrebs (Hirohasi, S., and Sugimura, T. Genetic alterations in human gastric cancer. Cancer cells (Cold Spring Habor), 3:49-52, 1991) und ERBB2 und MYC beim Ovarkarzinom (Sasano, H., Garrett, C.T., Wilkinson, D.S., Silverberg, S., Comerford, J., and Hyde, J. Protooncogene amplification and tumor ploidy in human ovarian neoplasm. Hum.Pathol., 21:382-391,1990) sehr schlecht. Beim Brustkrebs korreliert die Amplifikation von MYC (Borg, A., Baldetorp, B., Fernö, M., Olsson, H., and Sigurdsson, H. C-myc amplification is an independent prognostic factor in postmenopausal breast cancer. Int. J. Cancer, 51:687-691,1992) und Co-Amplifikation von INT2 und HST1 (Borg, A., Sigurdsson, H., Clark, G..M., et al., Association of INT2/HST1 coamplification in primary breast cancer with hormone-dependent phenotype and poor prognosis. Br.J.Cancer, 63:136-142, 1991) mit dem Krankheitsverlauf. Die Amplifikation von ERBB2 (Descotes, F., Pavy, J.-J., and Adessi, G.L. Human breast cancer: Correlation study between HER-2/neu amplification and prognostic factors in an unselected population. Anticancer Res., 13:119-124,1993) und EGFR (Klijn, J.G.M., Berns, P.M.J.J., Schmitz, P.I.M., and Foekens, J.A. The clinical significance of epidermal growth factor receptor (EGF-R) in human breast cancer: a review on 5232 patients. Endocr.Rev., 13:3-17,1992) ist mit einem schlechten Krankheitsverlauf verbunden. Beim Ösophaguskarzinom korreliert die Co-Amplifikation von HST1 und INT2 mit dem Grad der Metastasierung (Tsuda, T., Tahara, E., Kajiyama, G., Sakamoto, H., Terada, M., and Sugimura, T. High incidence of coamplification of hst-1 and int-2 genes in human esophageal carcinomas. Cancer Res. 49:5505-5508,1989).

Zusammenfassend kann festgestellt werden, daß durch chronische Behandlung mit kanzerogenen Zytostatika induzierte Genamplifikation nicht nur zur Resistenz gegenüber dieser Behandlung führt, sondern auch zur Überexpression gewisser Onkogene, welche den Grad der Malignität kontrollieren.

Es sind eine Reihe von Substanzen beschrieben worden, die der erworbenen Arzneimittelresistenz entgegenwirken. Hierzu gehören die von Kennedy (Kennedy, A.R., Prevention of Carcinogenesis by Protease Inhibitors, Cancer Res., 54:1999-2005,1994) beschriebenen Arbeiten über die anti-kanzerogenen Wirkungen von Protease-Inhibitoren. Diese können kanzerogeninduzierte Genamplifikation auf nahezu normale Level herunterdrücken. Kennedy beobachtete, daß strahleninduzierte Genamplifikation in gleicher Weise unterdrückt wird, wie es ihrer Fähigkeit entspricht, strahleninduzierte Transformation zu unterdrücken, so daß auf einen Zusammenhang zwischen beiden Prozessen geschlossen wird. Darüber hinaus wirken Protease-Inhibitoren als Antagonisten von (co-rekombinogenen) Tumor-Promotoren bei Auslösung von Transformation in vitro. Protease-Inhibitoren werden auch als wirkungsvolle Anti-Promotoren in In-vivo-Untersuchungen beschrieben (Troll, W., Klassen, A., and Janoff, A. Tumorigenesis in mouse skin: inhibition by synthetic inhibitors of proteases. Science (Washington DC) 169:1211-1213, 1970).

Aus der Literaturstelle Moscow, I.A., and Cowan, K.H. Multidrug resistance. J.Natl.Cancer Inst. 80: 14-20, 1988, ist bekannt, daß Verapamil der MDR entgegenwirkt. Dieser "caicium channel blocker" erhöht die Zytotoxizität durch Erhöhung der intrazellulären Akkumulation von Arzneimitteln. Wahrscheinlich geschieht dies durch eine Wirkung auf das P-Glykoprotein oder andere. Transportproteine. Die Toxizität dieser und ähnlicher Substanzen, wie z.B. Quinidin, steht ihrem klinischen Einsatz im Wege.

Ausgehend hiervon, ist es die Aufgabe der vorliegenden Erfindung, eine wirksame Substanz zur Verhinderung bzw. Reduzierung der Resistenzbildung gegenüber der Zytostatikabehandlung anzugeben.

Die Aufgabe wird durch die kennzeichnenden Merkmale des Anspruches 1 gelöst.

Erfindungsgemäß wird somit vorgeschlagen, die Resistenzbildung durch gleichzeitige Gabe von 5-substituierten Nukleosiden und einem Zytostatikum zu verhindern bzw. zu reduzieren. Es hat sich überraschenderweise gezeigt, daß 5-Nukleoside die Entstehung von kanzerogeninduzierten Genamplifikationen verhindert oder zumindest abschwächt. Dadurch wird die Möglichkeit eröffnet, durch gleichzeitige Gabe dieser Nukleoside mit einem Zytostatikum die Entstehung von Resistenzen gegenüber diesem Arzneimittel zu verhindern und auch den Grad der Malignität zu beeinflussen.

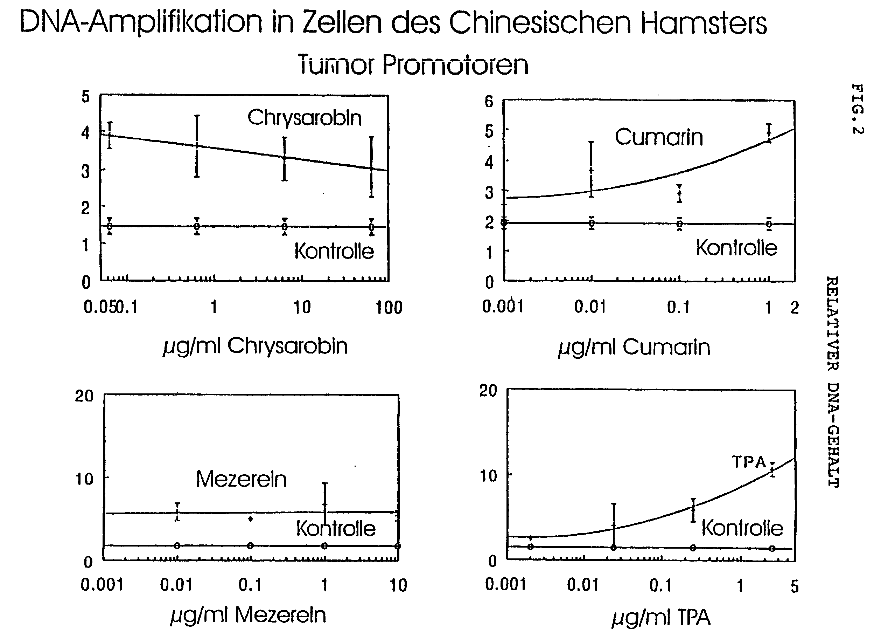

Als Beispiel für 5-Nukleoside sind zu nennen: 5-(2-bromovinyl-2'-deoxyuridin (BVDU), (E)-5-(2-bromovinyl)-1-β-D-arabinofuranosyluracil, (E)-5-(2-bromovinyl-2'-deoxy-4'-thiouridin. Abb. 2: 5-Jodo-2'-dexycytidin, 5-Jodo-2'-deoxyuridin, 2'-Deoxy-5-trifluoromethyluridin, besonders bevorzugt ist BVDU und (E)-5-(2-Bromovinyl-)uracyl (BVU).

Die 5-Nukleoside sind dabei in der Formulierung des Arzneimittels in einer Menge enthalten, aus der eine Konzentration von 0,02 µg/ml bis 10 µg/ml im Blut resultiert. In Versuchen konnte gezeigt werden, daß der optimale Bereich bei 0,05 µg/ml bis-5 µg/ml liegt.

Die Zytostatika können dabei in den üblichen bisher bekannten Mengen in der Formulierung enthalten sein (Oshiro, Y., Piper, C.E., Balwierz, P.S., and Garriot, M.L. (1992) Genotoxic properties of (E)-5-(2-bromovinyl)-2'-deoxyuridine (BVDU). Fundamental and Applied Toxicology, 18, 491-498). Beispiele für Zytostatika sind Cyclophosphamid und andere Alkylantien, Antimetabolite wie Methotrexat, Alkaloide wie Vinblastin, Antibiotika wie Bleomycin, Cisplatin sowie andere Stoffe. Weitere Beispiele für Zytostatika sind aus "Rote Liste 1995", Editio Cantor Verlag für Medizin und Naturwissenschaften, Aulendorf/Württ., 1995, zu entnehmen.

Die Träger-, Zusatz- und Hilfsstoffe entsprechen denen, wie sie bisher aus dem Stand der Technik bekannt sind. Das Arzneimittel selbst kann dabei in fester oder flüssiger Form oder auch als Spray vorliegen.

Die Erfindung wird nachfolgend anhand von Modellversuchen näher erläutert.

### A. Modellsubstanzen

Für die Untersuchung von Amplifikationsphänomenen dient eine Hamsterzellinie, die ein Virus (Simian Virus 40) im Genom integriert enthält. Bekannt ist für diese Zellinie, daß eine Zugabe genotoxischer Substanzen, aber auch verschiedener "nichtgenotoxischer" Kanzerogene und Tumor-Promotoren zur Amplifikation von SV40-DNA im Hamstergenom führt. Die Methode beruht darauf, daß als Sonde dienende markierte SV40-DNA mit Hamsterzell-DNA, die SV40-DNA in amplifizierter Kopienzahl enthält, hybridisiert wird. Die Menge gebundener Sonde gibt Aufschluß über den Amplifikationsgrad der integrierten Virus-DNA.

Zur Bestimmung des Amplifikationsgrades wird gleichzeitig mit der SV40-DNA die Albumin-Gen-DNA gemessen. Das Albumin-Gen wird nämlich im Gegensatz zur SV40-DNA in der Zelle nicht amplifiziert. Der Wert des relativen SV40-DNA-Gehalts ergibt sich demnach aus dem Quotienten des Signals der mit SV40-DNA hybridisierten DNA-Sonde zu dem Signal der mit Albumin-Gen hybridisierten DNA-Sonde aus denselben SV40-transformierten embryonalen CO631-Hamsterzellen.

Als Modellsubstanzen dienten:
1. Mutagene und rekombinogene genotoxische Kanzerogene (Positiv-Kontrolle)
   Triethylenmelamin (TEM)
   4-Nitrochinolin 1-Oxid (4-NQO)
2. Nicht-Kanzerogene (Negativ-Kontrolle), welche weder Mutationen noch Rekombinationen induzieren
   Aceton
   Dimethylformamid
3. Co-rekombinogene Tumor-Promotoren
   Mezerein
   12-O-tetradecanoyl-phorbol-13-acetat (TPA) Chrysarobin
   Coumarin
4. Rekombinogene nicht-genotoxische Kanzerogene mit unbekanntem Wirkmechanismus
   Thioacetamid
   Acetamid
5. Testosteron nach Metabolisierung durch Rattenlebermikrosomen (S9-Mix) sowie ohne S9-Mix
   Testosteron wirkt
   mit S9-Mix anti-rekombinogen und
   ohne S9-Mix co-rekombinogen

Die Wirkung der obengenannten Modellsubstanzen allein oder in Kombination mit einem Kanzerogen wurde im Genamplifikations-System untersucht.

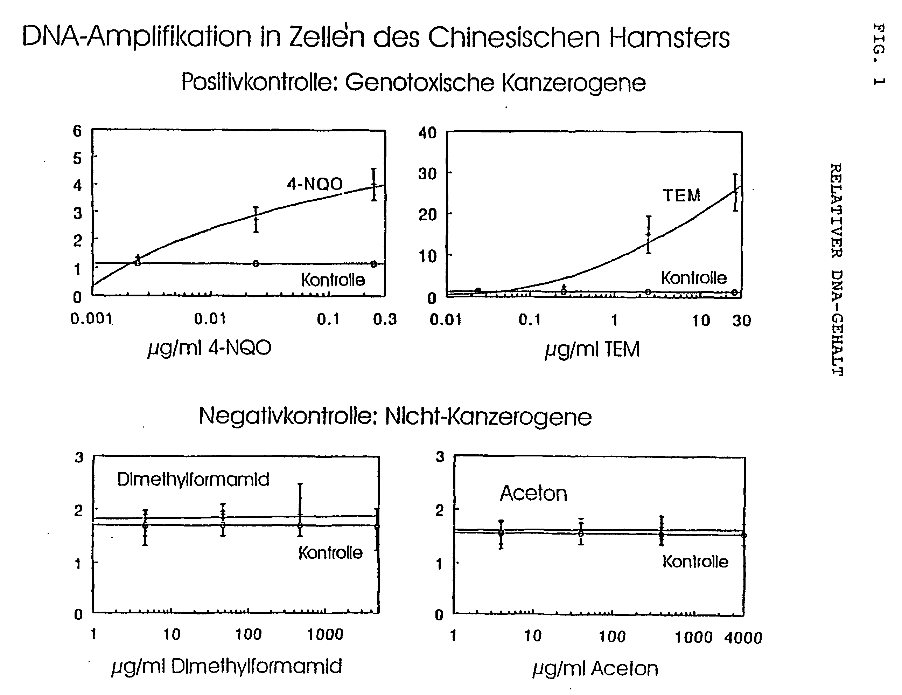

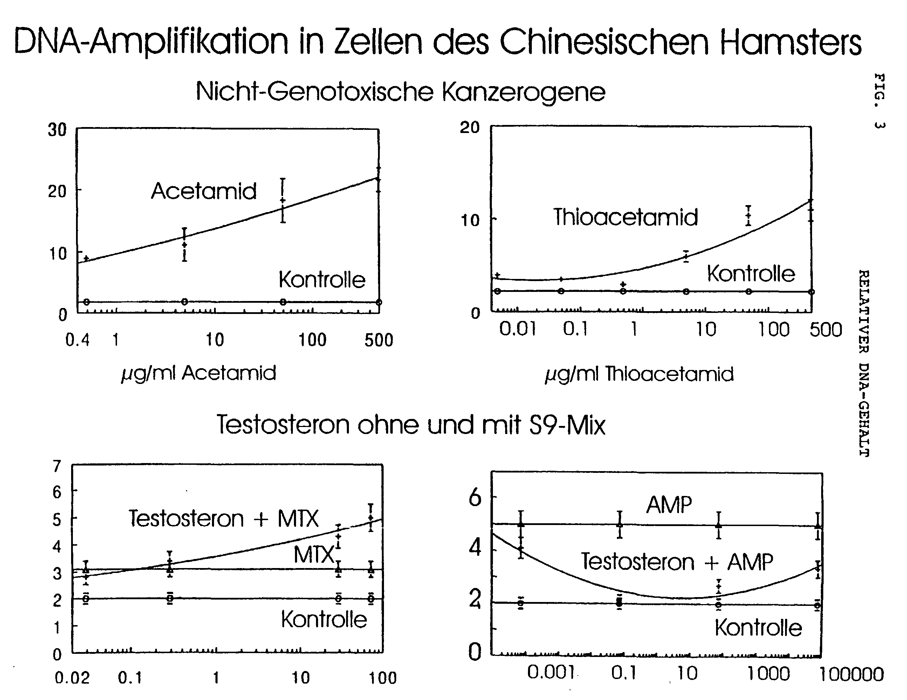

Die Ergebnisse mit den Modellsubstanzen sind in den Figuren 1 bis 3 zusammengefaßt.

Die Nicht-Kanzerogene Aceton und Dimethylformamid zeigen keine Wirkung.

Alle anderen Substanzen, die nicht-genotoxischen Kanzerogene mit unbekanntem Wirkmechanismus, Thioacetamid und Acetamid, die genotoxischen Kanzerogene TEM und 4-NQO sowie die Tumor-Promotoren Mezerein, 12-O-tetradecanoyl-phorbol-13-acetat (TPA), Chrysarobin und Coumarin, für sich allein gegeben, erhöhen die SV40-Genamplifikation.

Teststeron erniedrigt mit S9-Mix die amplifizierende Wirkung von Methotrexate (MTX), ohne S9-Mix erhöht es die amplifizierende Wirkung von Amino-6-mercaptopurin (AMP).

Diese Ergebnisse zeigen eine Übereinstimmung zwischen der Auslösung von Rekombination und SV40-Genamplifikation.

### B. Hemmung kanzerogeninduzierter Genamplifikation durch (E)-5-(2-Bromovinyl-)-2'-deoxyuridine (BVDU)

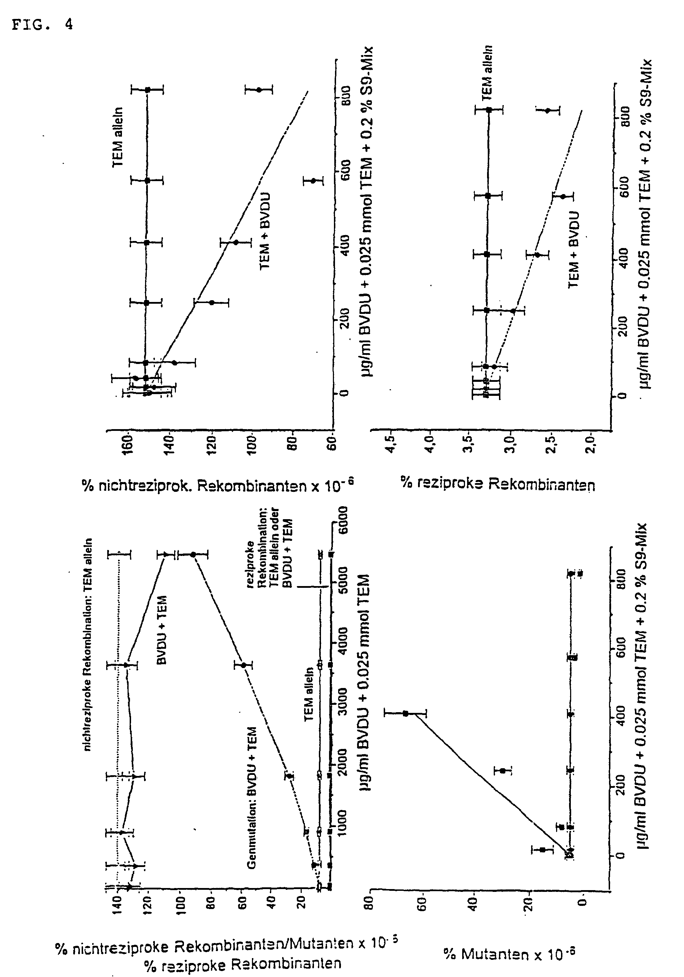

Die Ergebnisse sind in den Figuren 4 bis 7 zusammengefaßt.

BVDU hatte im Versuch mit Hefen (Figur 4) eine antirekombinogene und co-mutagene Wirkung gezeigt. Diese Wirkung war in Gegenwart von Lebermikrosomen (S9-Mix) in niedrigeren Konzentrationen sichtbarer als in Abwesenheit von S9-Mix und auch sehr viel ausgeprägter. Eine Metabolisierung von BVDU verstärkt also den antirekombinogenen Effekt.

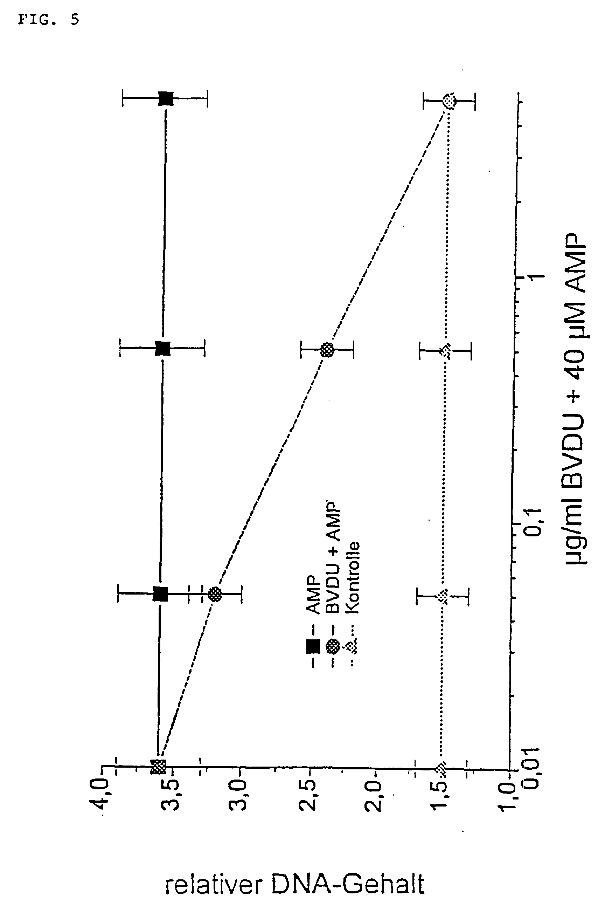

BDVU zeigt in klinisch relevanten Dosen eine Hemmung AMP-induzierter Genamplifikation. Der Effekt setzt bei etwa 0,05 µg/ml ein und führt dosisabhängig bei 5 µg/ml zu einer vollständigen Hemmung der AMP-induzierten Genamplifikation (Figur 5).

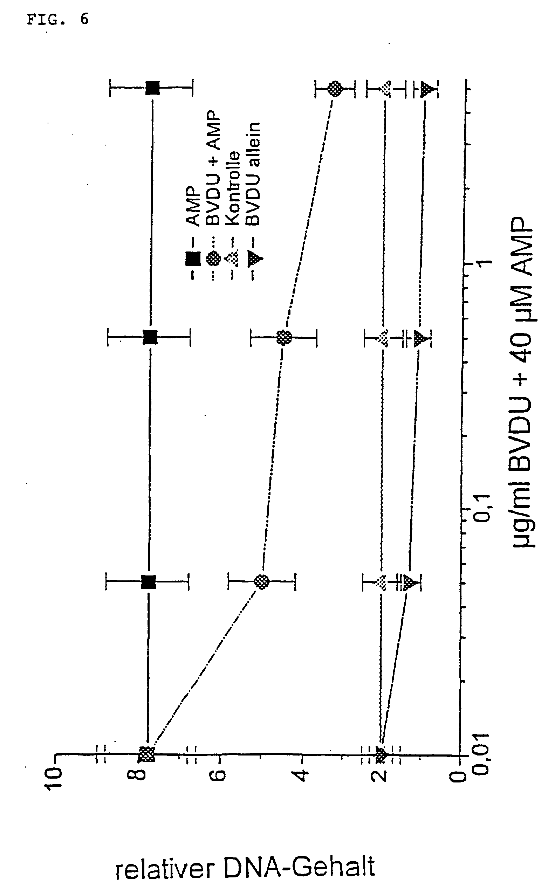

Der unabhängige Wiederholungsversuch bestätigt das Ergebnis (Figur 6). Darüber hinaus scheint BVDU allein den spontanen Amplifikationsgrad leicht zu erniedrigen.

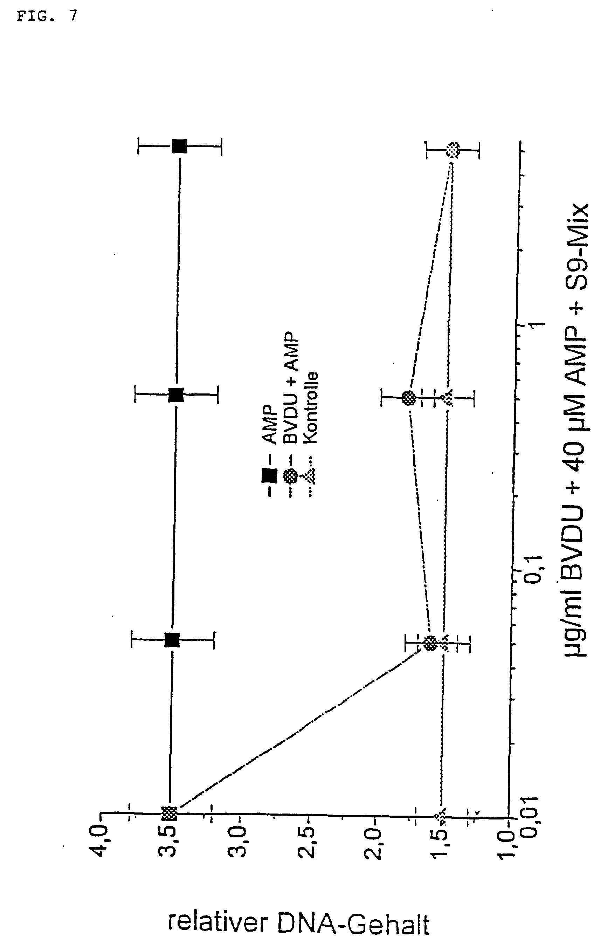

Zugabe von S9-Mix zeigt ebenfalls eine Erniedrigung AMP-induzierter Genamplifikation. Dieser erfolgt allerdings in einem niedrigeren Dosisbereich als in den Versuchen ohne S9-Mix. Eine mögliche Metabolisierung von BVDU scheint den amplifikationshemmenden Effekt also noch zu verstärken (Figur 7). Dies würde die Relevanz der Ergebnisse noch verstärken.

Zusammenfassend kann gesagt werden, daß BVDU kanzerogeninduzierte Genamplifikation hemmt. Dies eröffnet die Möglichkeit, durch gleichzeitige Gabe von BVDU mit einem Zytostatikum die Entstehung von Resistenzen gegenüber diesem Arzneimittel zu verhindern und die Malignität zu erniedrigen.

### C. Verhinderung der Bildung von "Multi-Drug-Resistance" (MDR) in menschlichen und tierischen Tumorzellen gegenüber Zytostatikabehandlung durch gleichzeitige Gabe von BVDU

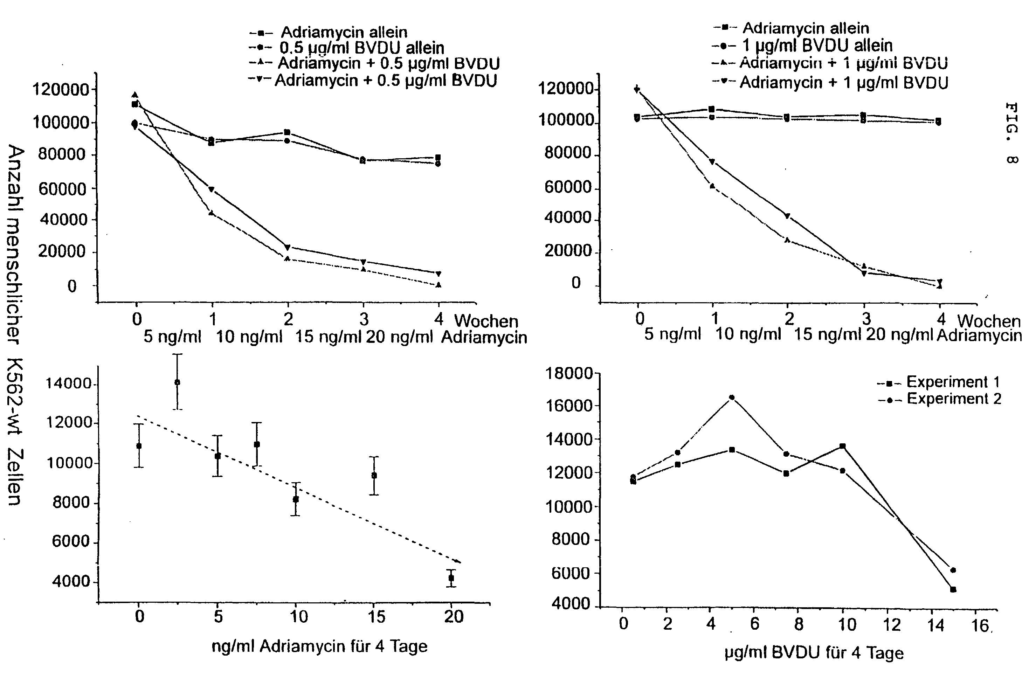

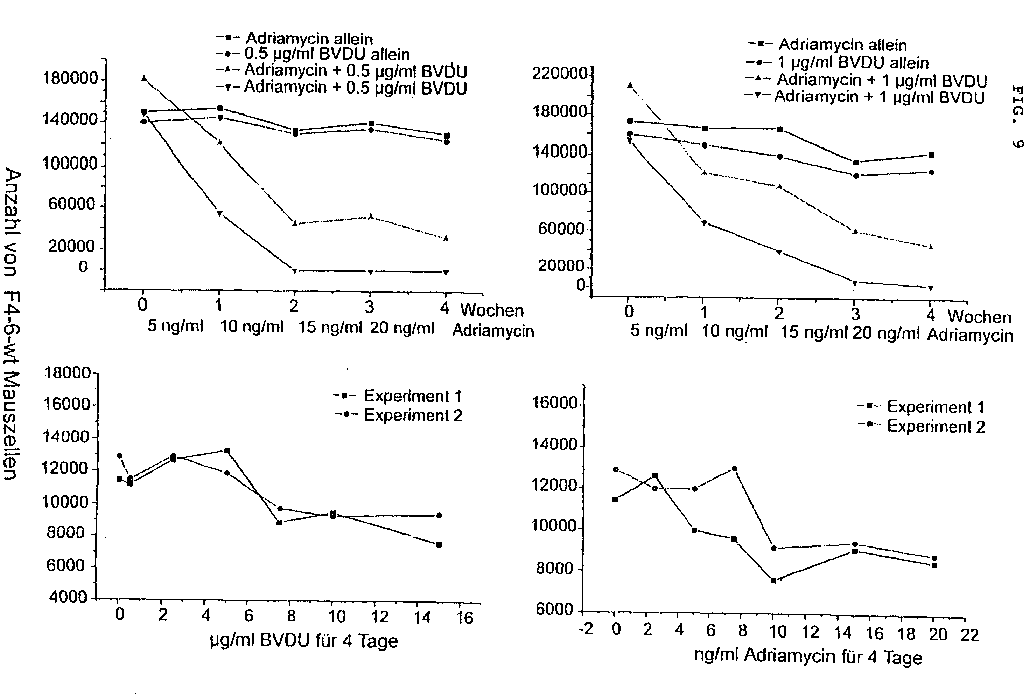

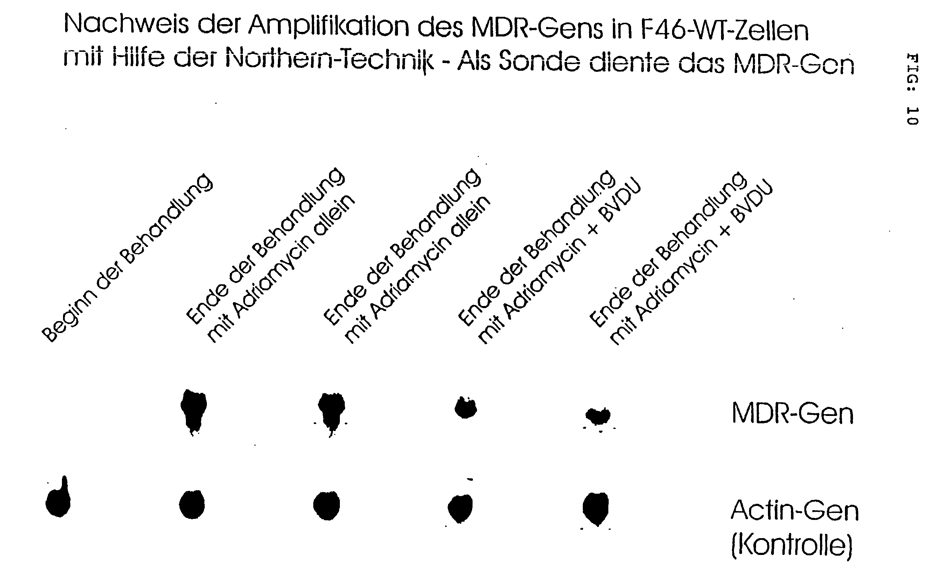

Die menschliche Tumorzellinie K562-WT (Fig. 8) und die Tumorzellinie F46-WT der Maus (Fig. 9) (WT = Wildtyp = empfindlich gegenüber Zytostatikabehandlung = keine Amplifikation des MDR-Gens) wurde über mehrere Wochen mit stufenweise steigenden Konzentrationen von Adriamycin behandelt. Im Laufe der Behandlung erwarben die Zellen eine Resistenz gegenüber dieser Behandlung. Wirken gegenüber nichtresistenten Zellen 20 ng/ml Adriamycin bei einer Behandlungszeit von 4 Tagen stark toxisch, so sind die Zellen nach Langzeitbehandlung mit stufenweise steigenden Konzentrationen gegenüber 20 ng/ml Adriamycin völlig unempfindlich geworden (Figuren 8 und 9). Die Resistenzbildung beruht auf der Amplifikation des MDR-Gens. Nachgewiesen wird dies mit Hilfe der Northern-Technik, eines Verfahrens zum Nachweis von RNA-Molekülen, also der Transkription eines Gens, unter Einsatz des MDR-Gens als Sonde (Figur 10). Resistente Zellen zeigen eine Bande, nichtresistente Zellen (Zustand am Beginn der Behandlung) zeigen keine Bande.

In Parallelversuchen wird Adriamycin mit entweder 0,5 oder 1 µg/ml BVDU zusammen gegeben (BDVU wirkt in menschlichen Tumorzellen erst ab etwa 10 µg/ml toxisch, in Zellen der Maus ab etwa 8 µg/ml (Figuren 8 und 9). BVDU verhindert die Resistenzbildung gegenüber Adriamycin. Die Tumorzellen bleiben empfindlich gegenüber der Zytostatikabehandlung und sterben ab. Die Wirkung des BVDU ist so stark, daß die Behandlung durch Ruhepausen (Wachstum ohne Substanzen) unterbrochen werden muß, sich der Versuch also über 6 bis 8 Wochen erstreckt.

BVDU + Adiamycin-Behandlung führt zu einer wesentlich schwächeren Amplifikation des MDR-Gens als Adriamycin-Behandlung allein (Figur 10). Der Effekt der BVDU-Behandlung ist in Wirklichkeit noch sehr viel größer, als die Abschwächung der Bande ausdrückt. Am Ende der Behandlung bleiben nämlich nur Zellen übrig, die wenigstens eine gewisse Resistenz gegenüber der Adriamycin-Behandlung erworben haben. Die infolge der BVDU-Behandlung nichtresistent gebliebenen Zellen sind bereits vorher abgestorben. Der eigentlich relevante und mit Hilfe der Northern-Technik nicht faßbare Effekt besteht deshalb im Absterben der nichtresistenten Zellen, welche in den Inaktivierungskurven (Figuren 8 und 9) gemessen wird.

Da die Resistenzbildung gegenüber Zytostatikabehandlung in menschlichen Tumoren ebenfalls auf der Amplifikation des MDR-Gens beruht, ist durch die Kombination von BVDU mit einem beliebigen Zytostatikum die Möglichkeit geboten, die Therapie in niedrigen Dosen und über längere Zeiträume als bisher durchzuführen. Im übrigen ist die Verhinderung der Resistenzbildung auch für andere Anwendungen von großer Bedeutung.

### D. Verhinderung der Bildung von "Multi-Drug-Resistance" (MDR) in Tumorzellen gegenüber Zytostatikabehandlung durch gleichzeitige Gabe von anti-rekombinogenen 5-substituierten Nukleosiden

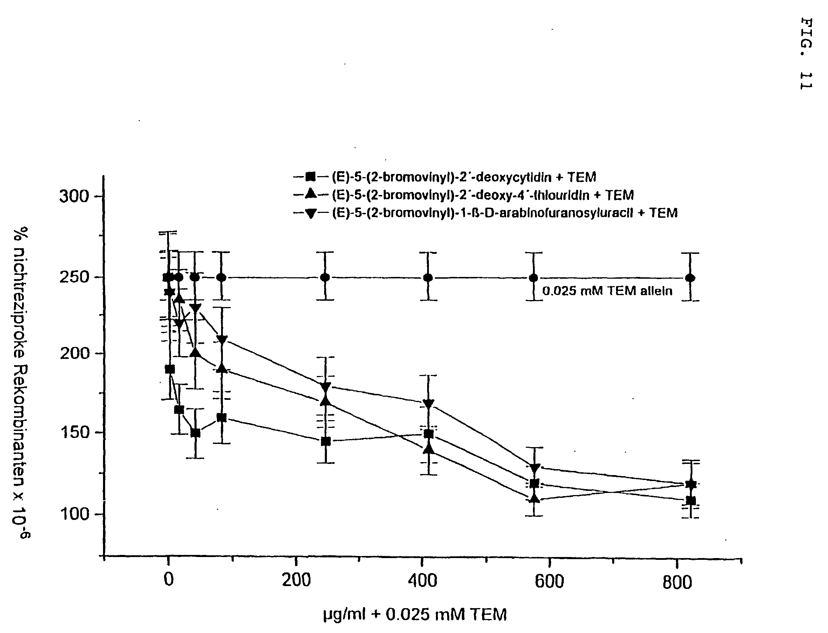

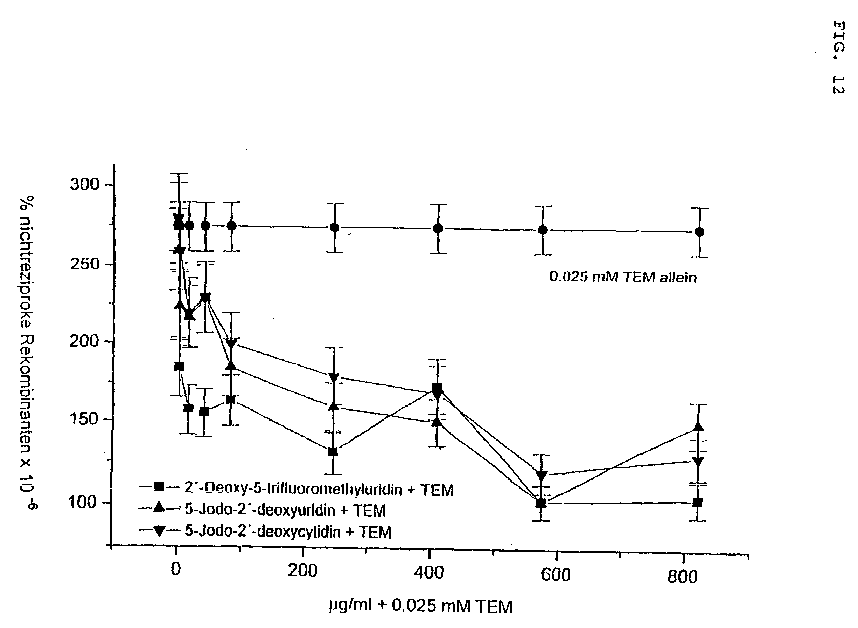

In den Figuren 11 und 12 ist zu sehen, daß die antirekombinogene Wirkung nicht nur spezifisch für BVDU gilt, sondern eine Eigenschaft aller 5-substituierten Nukleoside ist.

Fig. 11 zeigt dabei die anti-rekombinogenen Effekte von (E)-5-(2-bromovinyl)-2'-deoxyuridin, (E)-5-(2-bromovinyl)-1-β-D-arabinofuranosyl-uracil und (E)-5-(2-bromovinyl)-2'-deoxy-4'-thiouridin in Saccharomyces cerevisiae MP1, und
Fig. 12 die anti-rekombinogenen Effekte von 5-Jodo-2'-deoxycytidin, 5-Jodo-2'-deoxyuridin und 2'-Deoxy-5-trifluoromethyluridin in Saccharomyces cerevisiae MP1.

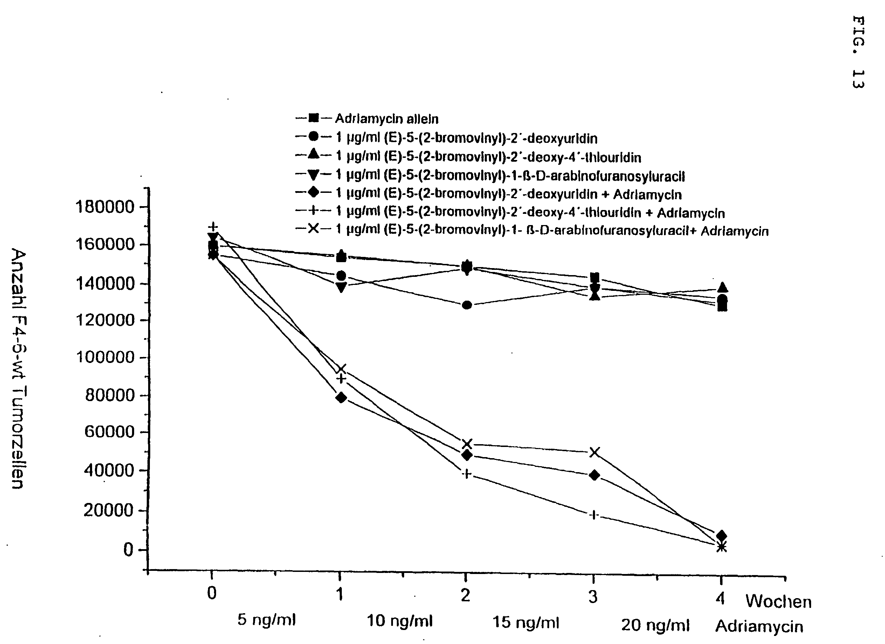

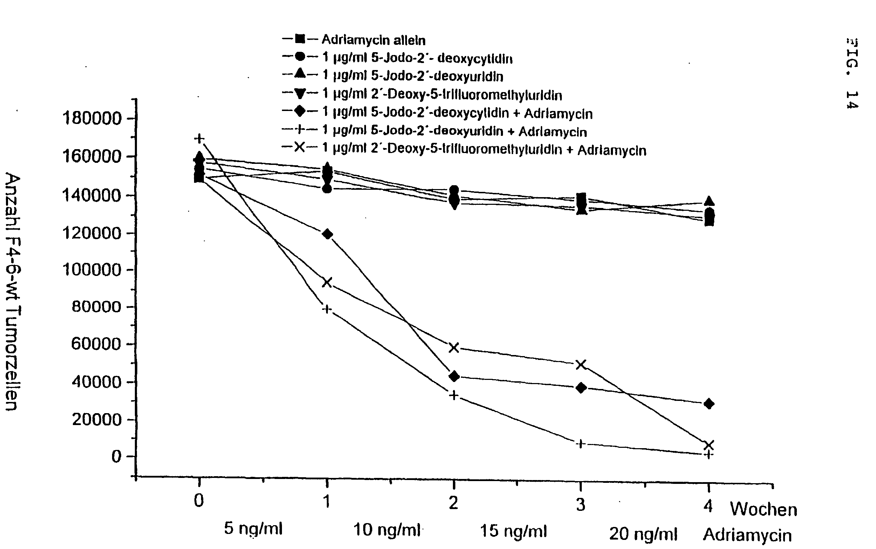

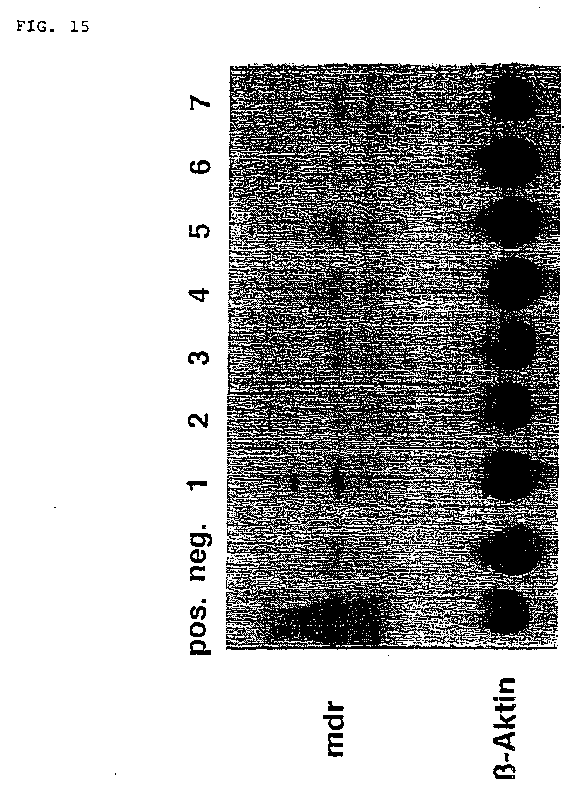

Die Tumorzellinie F4-6-WT der Maus (WT = Wildtyp = empfindlich gegenüber Zytostatikabehandlung = keine Amplifikation des MDR-Gens) wurde über mehrere Wochen mit stufenweise steigenden Konzentrationen von Adriamycin behandelt. Im Laufe der Behandlung erwarben die Zellen eine Resistenz gegenüber dieser Behandlung. Wirken gegenüber nichtresistenten Zellen 20 ng/ml Adriamycin bei einer Behandlungszeit von 4 Tagen stark toxisch, so sind die Zellen nach Langzeitbehandlung mit stufenweise steigenden Konzentrationen gegenüber 20 ng/ml Adriamycin völlig unempfindlich geworden (Figur 13 und 14). Die Resistenzbildungberuht auf der Amplifikation des MDR-Gens. Nachgewiesen wurde dies mit Hilfe der Northern-Technik, eines Verfahrens zum Nachweis von RNA-Molekülen, also der Transkription eines Gens, unter Einsatz des MDR-Gens als Sonde (Figur 15). Resistente Zellen zeigen eine Bande, nichtresistente Zellen (Zustand am Beginn der Behandlung) zeigen keine Bande. Die Level von β-Actin mRNA wurden vergleichsweise ebenfalls analysiert. β-Actin wurde als interne Kontrolle für die RNA-Menge benutzt.

In Parallelversuchen wurde Adriamycin mit 1 µg/ml je eines 5-substituierten Nukleosids zusammen gegeben. Alle sechs 5-substituierten Nukleoside verhindern die Resistenzbildung gegenüber Adriamycin. Die Tumorzellen bleiben empfindlich gegenüber der Zytostatikabehandlung und sterben ab. Die Wirkung der 5-substituierten Nukleoside war so stark, daß die Behandlung durch Ruhepausen (Wachstum ohne Substanzen) unterbrochen werden mußte, sich der Versuch also über 6 bis 8 Wochen erstreckte.

Figur 15 zeigt die Northern Blot Analyse der RNA: Expression der MDR-Gene in der Tumorzellinie F4-6-WT der Maus. Die Level von β-Actin mRNA wurden vergleichsweise ebenfalls analysiert. β-Actin wurde als interne Kontrolle für die RNA-Menge benutzt. pos. Adriamycin-resistente F4-6-WT Zellen neg. Adriamycin-empfindliche F4-6-WT Zellen
1 1 µg/ml (E)-5-(2-bromovinyl)-2'-deoxyuridin + Adriamycin
2 1 µg/ml (E)-5-(2-bromovinyl-1-β-D-arabinofuranosyl-uracil + Adriamycin
3 1 µg/ml (e)-5-(2-bromovinyl-2'-deoxy-4'-thiouridin + Adriamycin
4 1 µg/ml 5-Jodo-2'-deoxycytidin + Adriamycin
5 1 µg/ml 5-Jodo-2'-deoxyuridin + Adriamycin
6 1 mg/ml 2'-Deoxy-5-trifluoromethyluridin + Adriamycin
7 1 µg/ml (E)-5-(2-bromovinyl-)-2'-deoxyuridine (BVDU) + Adriamycin

5-substituiertes Nukleosid + Adriamycin-Behandlung führt zu einer wesentlich schwächeren Amplifikation des MDR-Gens als Adriamycin-Behandlung allein (Figur 15). Der Effekt der Behandlung ist in Wirklichkeit noch sehr viel größer als es die Abschwächung der Bande ausdrückt. Am Ende der Behandlung bleiben nämlich nur Zellen übrig, die wenigstens eine gewisse Resistenz gegenüber der Adriamycin-Behandlung erworben haben. Die infolge der BVDU-Behandlung nichtresistent gebliebenen Zellen sind ja bereits vorher abgestorben. Der eigentlich relevante und mit Hilfe der Northern-Technik nicht faßbare Effekt besteht deshalb im Absterben der nichtresistenten Zellen, welcher in den Inaktivierungskurven (Figur 13 und 14) gemessen wurde.

## Patentansprüche

1. Verwendung von 5-substituierten Nukleosiden in Kombination mit mindestens einem Zytostatikum zur Herstellung eines Arzneimittels zur Verhinderung bzw. Reduzierung der Resistenzenbildung bei der Zytostatikabehandlung.

2. Verwendung nach Anspruch 1,
**dadurch gekennzeichnet, daß** als Nukleosid (E)-5-(2-Bromovinyl-)-2'-deoxyuridin (BVDU) verwendet wird.

3. Verwendung nach Anspruch 2,
**dadurch gekennzeichnet, daß** als Metabolit (E)-5-(2-Bromovinyl-)-uracyl (BVU) verwendet wird.

4. Verwendung nach Anspruch 1,
**dadurch gekennzeichnet, daß** als Nukleosid (E)-5-(2-bromovinyl)-1-β-D-arabinofuranosyluracil verwendet wird.

5. Verwendung nach Anspruch 1,
**dadurch gekennzeichnet, daß** als Nukleosid (E)-5-(2-bromovinyl) -2'-deoxy-4'-thiouridin verwendet wird.

6. Verwendung nach Anspruch 1,
**dadurch gekennzeichnet, daß** als Nukleosid 5-Jodo-2'-deoxycytidin verwendet wird.

7. Verwendung nach Anspruch 1,
**dadurch gekennzeichnet, daß** als Nukleosid 5-Jodo-2'-deoxyuridin verwendet wird.

8. Verwendung nach Anspruch 1,
**dadurch gekennzeichnet, daß** als Nukleosid 2'-Deoxy-5-trifluoromethyluridin verwendet wird.

## Claims

1. Use of 5-substituted nucleosides in combination with at least one cytostaticum for producing a pharmaceutical for prevention or reduction of development of resistance during cytostatica treatment.

2. Use according to Claim 1, **characterised in that** (E)-5-(2-bromovinyl-)-2'-deoxyuridine (BVDU) is used as nucleoside.

3. Use according to Claim 2, **characterised in that** (E)-5-(2-bromovinyl-)-uracyl (BVU) is used as metabolite.

4. Use according to Claim 1, **characterised in that** (E)-5-(2-bromovinyl)-1-β-D-arabinofuranosyl uracil is used as nucleoside.

5. Use according to Claim 1, **characterised in that** (E)-5-(2-bromovinyl)-2'-deoxy-4'-thiouridine is used as nucleoside.

6. Use according to Claim 1, **characterised in that** 5-iodo-2'-deoxycytidine is used as nucleoside.

7. Use according to Claim 1, **characterised in that** 5-iodo-2'-deoxy uridine is used as nucleoside.

8. Use according to Claim 1, **characterised in that** 2'-deoxy-5-trifluoromethyl uridine is used as nucleoside.

## Revendications

1. Utilisation de nucléosides substitués en position 5 en combinaison avec au moins un cytostatique pour la fabrication d'un médicament évitant ou réduisant la formation de résistances lors du traitement phytostatique.

2. Utilisation selon la revendication 1, **caractérisée en ce que** l'on utilise comme nucléoside le (E)-5-(2-bromovinyle)-2'-désoxy uridine (BVDU).

3. Utilisation selon la revendication 2, **caractérisée en ce que** l'on utilise comme métabolite le (E)-5-(2-bromovinyle)-uracile (BVU).

4. Utilisation selon la revendication 1, **caractérisée en ce que** l'on utilise comme nucléoside le (E)-5-(2-bromovinyle)-1-β-D-arabinofuranosyle uracile.

5. Utilisation selon la revendication 1, **caractérisée en ce que** l'on utilise comme nucléoside le (E)-5(2-bromovinyle)-2'-désoxy-4'-thio-uridine.

6. Utilisation selon la revendication 1, **caractérisée en ce que** l'on utilise comme nucléoside le 5-iodo-2'-désoxy cytidine.

7. Utilisation selon la revendication 1, **caractérisée en ce que** l'on utilise comme nucléoside le 5-iodo-2'-désoxyuridine.

8. Utilisation selon la revendication 1, **caractérisée en ce que** l'on utilise comme nucléoside le 2'-désoxy-5-trifluorométhyle uridine.
